Europäisches Patentamt
European Patent Office
Office européen des brevets

(19)

(11) Publication number: **0 322 251 A2**

(12) **EUROPEAN PATENT APPLICATION**

(21) Application number: **88312288.9**

(22) Date of filing: **23.12.88**

(51) Int. Cl.4: **C 07 D 409/04**
C 07 D 335/06,
C 07 D 495/04, A 61 K 31/38,
A 61 K 31/445, A 61 K 31/40

(30) Priority: **23.12.87 GB 8730052**

(43) Date of publication of application:
**28.06.89 Bulletin 89/26**

(84) Designated Contracting States:
**BE CH DE FR GB IT LI NL**

(71) Applicant: **BEECHAM GROUP PLC**
**Beecham House Great West Road**
**Brentford Middlesex TW8 9BD (GB)**

(72) Inventor: **Smith, David Glynn Beecham Pharmaceuticals**
**Great Burgh Yew Tree Bottom Road**
**Epsom Surrey KT18 5XQ (GB)**

**Stemp, Geoffrey Beecham Pharmaceuticals**
**Coldharbour Road The Pinnacles**
**Harlow Essex CM19 5AD (GB)**

(74) Representative: **Rutter, Keith et al**
**Beecham Pharmaceuticals Great Burgh Yew Tree**
**Bottom Road**
**Epsom Surrey KT18 5XQ (GB)**

The title of the invention has been amended (Guidelines for Examination in the EPO, A-III, 7.3).

(54) **Benzothiopyran derivatives.**

(57) A compound of formula (I):

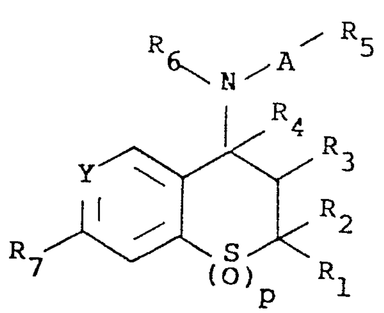

(I)

or, where appropriate, a pharmaceutically acceptable salt thereof, or a pharmaceutically acceptable solvate thereof wherein:
A represents >C=X, wherein X represents oxygen or sulphur, or A represents a bond;

Y represents N or $N^+-O^-$ or a moiety $CR_8$ wherein $R_8$ is as defined below;
$R_1$ and $R_2$ independently represent hydrogen or alkyl; or $R_1$ and $R_2$ together represent a $C_{2-7}$ polymethylene moiety;
$R_3$ represents hydrogen, hydroxy, alkoxy or acyloxy and $R_4$ is hydrogen or $R_3$ and $R_4$ together represent a bond;
when A represents >C=X, then $R_5$ is hydrogen; alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_6$ represents hydrogen or alkyl; or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstituted methylene group, $A^2$ represents 2 or 3 linking members, one of the linking members optionally

EP 0 322 251 A2

representing O, S or NR and the other linking members each independently representing a substituted or unsubstituted methylene group; R represents hydrogen, alkyl, alkanoyl, phenyl $C_{1-4}$-alkyl, arylcarbonyl wherein the aryl group may be substituted or unsubstituted; or R is mono- or bi-cyclic-heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted; $R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d$ (OH)CH-, $R^d$(SH)CH-, $R^dC(=N.OH)$-, $R^dC(=N.NH_2)$- or alkenyl optionally substituted by alkylcarbonyl, nitro or cyano, providing that when Y is $CR_8$ then at least one of $R_7$ or $R_8$ is not hydrogen;

$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;

$R^b$ and $R^c$ each independently represent hydrogen, alkyl or alkylcarbonyl;

T represents a bond or $T^1$, $T^1$ represents -CS- or $T^2$ and $T^2$ represents -CO-, -SO- or $-SO_2$-; and

p represents zero or an integer 2; a process for preparing such a compound, a pharmaceutical composition containing such a compound and the use of such a compound or composition in medicine.

**Description**

## NOVEL COMPOUNDS

This invention relates to certain novel compounds, in particular to novel benzothiopyran derivatives having smooth muscle relaxant activity, to processes for their preparation, to compositions containing such compounds and to the use of such compounds and compositions in medicine.

EP-A-76075, 93535, 95316, 107423, 120426, 126311, 126350, 126367 and 138134 describe certain benzopyran derivatives having inter alia antihypertensive activity. EP-A-176689 also discloses that certain benzopyran derivatives are useful for the treatment of inter alia disorders of the respiratory system. United Kingdom Patent Number 2145720 also discloses certain thioflavene derivatives having inter alia activity as relaxants for bronchial smooth muscle.

A group of novel benzothiopyran derivatives has now been discovered which surprisingly has smooth muscle relaxant activity, and such compounds are therefore potentially useful as bronchodilators in the treatment of disorders of the respiratory tract, such as reversible airways obstruction and asthma, and also in the treatment of hypertension. Such compounds are also indicated to be of potential use in the treatment of disorders associated with smooth muscle contraction of the gastro-intestinal tract, uterus or the urinary tract including the ureter. Such disorders respectively include irritable bowel syndrome and diverticular disease; premature labour; incontinence; renal cholic and disorders associated with the passage of kidney stones. They are also indicated as of potential use in the treatment of cardiovascular disorders other than hypertension, such as congestive heart failure, angina, peripheral vascular disease and cerebral vascular disease; and also in the treatment and/or prophylaxis of disorders associated with pulmonary hypertension and of disorders associated with right heart failure.

Accordingly, the present invention provides a compound of formula (I):

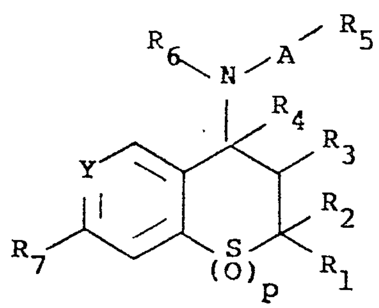

(I)

or, where appropriate, a pharmaceutically acceptable salt thereof, or a pharmaceutically acceptable solvate thereof

wherein:
A represents >C=X, wherein
X represents oxygen or sulphur, or A represents a bond;

Y represents N or $N^+O^-$ or a moiety $CR_8$ wherein
$R_8$ is as defined below;
$R_1$ and $R_2$ independently represent hydrogen or alkyl; or
$R_1$ and $R_2$ together represent a $C_{2-7}$ polymethylene moiety;
$R_3$ represents hydrogen, hydroxy, alkoxy or acyloxy and $R_4$ is hydrogen or $R_3$ and $R_4$ together represent a bond;
when A represents >C=X, then $R_5$ is hydrogen;
alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_6$ represents hydrogen or alkyl;
or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstituted methylene group, $A^2$ represents 2 or 3 linking members, one of the linking members optionally representing O, S or NR and the other linking members each independently representing

a substituted or unsubstituted methylene group; R represents hydrogen, alkyl, alkanoyl, phenyl $C_{1-4}$-alkyl, arylcarbonyl wherein the aryl group may be substituted or unsubstituted; or R is mono- or bi-cyclic-heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted;

$R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(=N.NH_2)$- or alkenyl optionally substituted by alkylcarbonyl, nitro or cyano, providing that when Y is $CR_8$ then at least one of $R_7$ or $R_8$ is not hydrogen;

$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;

$R^b$ and $R^c$ each independently represent hydrogen, alkyl or alkylcarbonyl;

T represents a bond or $T^1$, $T^1$ represents -CS- or $T^2$ and

$T^2$ represents -CO-, -SO- or $-SO_2$-; and

p represents zero or an integer 2.

Suitable substituents for any aryl or heteroaryl group represented by $R_5$ include one or more groups or atoms selected from alkyl, alkoxy, hydroxy, halogen, fluoroalkyl, nitro, cyano, carboxy or an ester thereof, alkylcarbonyloxy, amino, monoalkylamino, dialkylamino, aminocarbonyl, monoalkylaminocarbonyl or dialkylaminocarbonyl.

When the linking chain $-A^1-A^2-$ comprises substituted methylene groups it is favoured if one or two of methylene groups are substituted, in particular it is favoured if the methylene group represented by $-A^1-$ is substituted.

Suitable substituents for any methylene group in $-A^1-A^2-$ include alkyl groups, especially methyl or ethyl and in particular methyl.

In one particular aspect when A represents $>C=X$, the linking chain $-A^1-A^2-$ (and thus $R_5$ and $R_6$ together) represent a moiety of formula $-CH_2-(CH_2)_n-Z-(CH_2)_m-$wherein m and n are 0 to 2 such that m + n is 1 or 2 and Z is $CH_2$, O, S or NR wherein R is as defined above.

Suitably R represents hydrogen, $C_{1-9}$ alkyl, $C_{2-7}$ alkanoyl, phenyl-$C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl.

Suitably, when Y is N or $N^+-O^-$, $R_7$ is hydrogen.

Suitably, when Y is $CR_8$, then one of $R_7$ and $R_8$ is hydrogen.

In particular, when Y is $CR_8$, one of $R_7$ and $R_8$ is hydrogen and the other is selected from substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(N.OH)$-, $R^dC(=N.NH_2)$- or alkenyl optionally substituted by alkylcarbonyl, nitro or cyano, wherein $R^a$, $R^b$, $R^c$, $R^d$, T, $T^1$ and $T^2$ are as defined in relation to formula (I).

Also to be mentioned are those compounds wherein one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxythiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl)NOH or $-C(C_{1-6}$ alkyl)NNH2.

In a further particular aspect, $R_7$ represents hydrogen and $R_8$ represents alkyl or $C_{3-8}$ cycloalkyl, especially alkyl.

Suitably, one of $R_7$ and $R_8$ represents nitro, cyano or alkylcarbonyl and the other represents a different group selected from nitro, cyano, halo, alkylcarbonyl, alkoxy, especially methoxy, mono- or di-alkylamino or mono- or di- alkylcarbonylamino. For example, one of $R_7$ and $R_8$ may represent nitro, cyano or alkylcarbonyl and the other may represent alkoxy, especially methoxy, amino, mono- or di-alkylamino or mono- or di-alkylcarbonylamino.

When $R_7$ or $R_8$ represents a substituted alkyl group, suitable optional substituents include one or more of halogen, hydroxy, alkoxy, thiol, nitro, cyano and alkylcarbonyl, especially halogen.

Preferably, when A represents $>C=X$ then X is oxygen.

In one particular aspect, the present invention provides a compound of formula (I), or, where appropriate, a pharmaceutically acceptable salt thereof, or a pharmaceutically acceptable solvate thereof
wherein:

X represents oxygen or sulphur;

Y represents N or $N^+-O^-$ or a moiety $CR_8$ wherein $R_8$ is as defined below;

$R^1$ and $R^2$ independently represent hydrogen or $C_{1-6}$ alkyl; or $R_1$ and $R_2$ together represent a $C_{2-7}$ polymethylene moiety;
$R_3$ represents hydrogen, hydroxy, $C_{1-6}$ alkoxy or $C_{1-7}$ acyloxy and $R_4$ is hydrogen or $R_3$ and $R_4$ together represent a bond;
$R_5$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or $C_{2-6}$ alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and $R_6$ represents hydrogen or $C_{1-6}$ alkyl;
or $R_5$ and $R_6$ together represent $-CH_2-(CH_2)_n-Z-(CH_2)_m-$wherein m and n are O to 2 such that m + n is 1 or 2 and Z is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl;

when Y is N or $N^+-O^-$, $R_7$ is hydrogen or, when Y is $CR_8$, either one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxythiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl)NOH or $-C(C_{1-6}$ alkyl)$NNH_2$; or one of $R_7$ and $R_8$ is nitro, cyano or $C_{1-3}$ alkylcarbonyl and the other is methoxy or amino optionally substituted by one or two $C_{1-6}$ alkyl or by $C_{2-7}$ alkanoyl; or $R_7$ is hydrogen and $R_8$ is $C_{1-6}$ alkyl or $C_{3-8}$ cycloalkyl; and
p represents zero or an integer 2; suitably when $R_3$ represents hydroxy, alkoxy, in particular $C_{1-6}$ alkoxy, or acyloxy, in particular $C_{1-7}$ acyloxy, and $R_4$ is hydrogen, then the moiety $R_6$.N.CX.$R_5$ is trans to $R_3$.

Preferably, Y represents C-$R_8$ wherein $R_8$ is as defined in relation to formula (I).

Preferably, $R_1$ and $R_2$ are both $C_{1-6}$ alkyl, and in particular $R_1$ and $R_2$ are both methyl.

When $R_3$ is alkoxy, suitably $C_{1-6}$ alkoxy, and $R_4$ is hydrogen, preferred examples of $R_3$ include methoxy and ethoxy, of which methoxy is more preferred. When $R_3$ is acyloxy, suitably $C_{1-7}$ acyloxy, and $R_4$ is hydrogen, a preferred class of $R_3$ is unsubstituted carboxylic acyloxy, such as unsubstituted aliphatic acyloxy. However, it is more preferred that $R_3$ and $R_4$ together are a bond, or that $R_3$ and $R_4$ are both hydrogen, or in particular, that $R_3$ is hydroxy and $R_4$ is hydrogen.

When $R_5$ represents alkyl it is suitably $C_{1-6}$ alkyl, favoured alkyl groups include methyl, ethyl and n- and iso-propyl, preferably methyl.

A suitable halogen substituent for any alkyl represented by $R_5$, is a fluoro, chloro or bromo substituent; favoured examples include methyl, ethyl or propyl, especially n-propyl, terminally substituted by fluoro, chloro or bromo, for example chloro.

When $R_5$ represents alkyl substituted by hydroxy, favoured examples include methyl or ethyl terminally substituted by hydroxy.

When $R_5$ represents alkyl substituted by alkoxy, a suitable alkoxy group is a methoxy or ethoxy group; favoured examples include methyl or ethyl terminally substituted by methoxy or ethoxy.

When $R_5$ represents alkyl substituted by alkoxycarbonyl, suitably $C_{1-6}$ alkoxycarbonyl, a suitable alkoxycarbonyl group is a methoxycarbonyl or ethoxycarbonyl group; examples include methyl or ethyl terminally substituted by methoxycarbonyl or ethoxycarbonyl.

When $R_5$ represents alkyl substituted by carboxy, favoured examples include methyl or ethyl terminally substituted by carboxy.

When $R_5$ represents alkyl substituted by amino wherein the amino group is optionally substituted by one or two independent alkyl groups, favoured values include a group $(CH_2)_nNR_9R_9{}^1$ where n is 1 to 6, and $R_9$ and $R_9{}^1$ are each independently hydrogen or $C_{1-6}$ alkyl; suitable values for n include 1 and 2, in particular 1.

Preferably $R_9$ and $R_9{}^1$ are each independently selected from hydrogen and methyl.

When $R_5$ represents alkenyl, suitably $C_{2-6}$ alkenyl, suitable values include vinyl, prop-1-enyl, prop-2-enyl, 1-methylvinyl, but-1-enyl, but-2-enyl, but-3-enyl, 1-methylenepropyl, or 1-methylprop-2-enyl, in both their E and Z forms where stereoisomerism exists.

When $R_5$ represents optionally substituted amino, suitable optional substituents for the amino group include a methyl; ethyl; propyl; butyl; allyl or a trichloroacetyl group; or a phenyl group optionally substituted by one methyl, methoxy group or one halogen atom, and in particular a phenyl group optionally substituted with amino, methylamino or phenylamino; the phenyl group in the phenylamino substituent being optionally substituted in the phenyl ring by one methyl or methoxy group or one halogen atom.

When $R_5$ represents aryl, favoured examples include phenyl and naphthyl, preferably phenyl.

When $R_5$ represents heteroaryl, suitable heteroaryl groups include 5- or 6-membered monocyclic or 9- or

10-membered bicyclic heteroaryl groups, preferably 5-or 6-membered monocyclic heteroaryl groups.

Preferred 5- or 6-membered monocyclic or 9- or 10-membered bicyclic heteroaryl groups are those containing one, two or three heteroatoms selected from the class of oxygen, nitrogen and sulphur and which, in the case of there being more than one heteroatom, are the same or different.

Suitable 5- or 6-membered monocyclic heteroaryl moieties include furyl, thienyl, pyrrolyl, oxazolyl, thiazolyl, imidazolyl and thiadiazolyl, and pyridyl, pyridazyl, pyrimidyl, pyrazyl and triazyl. Preferred 5- or 6- membered heteroaryl groups include furyl, thienyl, pyrrolyl and pyridyl, in particular 2- and 3-furyl, 2- and 3-pyrrolyl 2- and 3-thienyl, and 2-, 3-and 4-pyridyl.

Suitable 9- or 10-membered bicyclic heteroaryl moieties include benzofuryl, benzothienyl, indolyl and indazolyl, quinolinyl and isoquinolinyl, and quinazolinyl. Preferred 9- or 10- membered bicyclic heteroaryl groups include 2- and 3-benzofuryl, 2- and 3-benzothienyl, and 2- and 3-indolyl, and 2- and 3-quinolinyl.

In any optionally substituted aryl or optionally substituted heteroaryl group, the preferred number of substituents is 1, 2, 3 or 4.

Preferred substituents for any substituted aryl or heteroaryl group include methyl, methoxy, hydroxy, chloro, fluoro, nitro or cyano.

One preferred sub-group of values for $R_5$ is that wherein $R_5$ represents phenyl or naphthyl or a 5- or 6-membered monocyclic or a 9- or 10-membered bicyclic heteroaryl, the phenyl, naphthyl or heteroaryl group being optionally substituted by one, two, three or four groups or atoms selected from the class of alkyl, alkoxy, halogen, fluoroalkyl, nitro or cyano.

When $R_5$ represents optionally substituted phenyl, preferred values include phenyl, 4-substituted phenyl, 3-substituted phenyl, 3,4-disubstituted phenyl and 3, 4, 5-trisubstituted phenyl, for example $R_5$ may suitably represent 4-fluorophenyl.

When $R_5$ represents an optionally substituted 5- or 6-membered monocyclic heteroaryl or an optionally substituted 9- or 10-membered bicyclic heteroaryl group, preferred values include unsubstituted 5- or 6-membered monocyclic heteroaryl or mono-substituted 5-or 6-membered monocyclic heteroaryl or 9- or 10-membered bicyclic heteroaryl, in particular unsubstituted 5- or 6-membered monocyclic heteroaryl or 9- or 10-membered bicyclic heteroaryl.

Preferably, when $R_5$ and $R_6$ together represent a linking chain $-A^1-A^2-$, $A^1$ represents a substituted or unsubstituted methylene group and $A^2$ represents a $-CH_2CH_2-$ or $-CH_2CH_2CH_2-$ group, for example $-CH_2CH_2-$.

When $A^1$ represents a substituted methylene group it is preferably substituted by an alkyl group especially a methyl group.

In a preferred aspect $R_5$ and $R_6$ together represent the moiety $-CH_2-(CH_2)_n-Z-(CH_2)_m-$as hereinbefore defined and the moiety $R_5$.N.CX.$R_6$ represents either pyrrolidonyl or piperidonyl.

When Z is other than $CH_2$, m is often 0 or 1 and n is often 0 or 1.

When Z represents NR, suitable arylcarbonyl groups include naphthylcarbonyl, phenylcarbonyl or benzylcarbonyl optionally substituted in the phenyl or naphthyl ring by one or two of alkyl, alkoxy or halogen.

Suitable examples of R when Z is NR include hydrogen, methyl, ethyl, n- and iso-propyl, n-, sec- and tert-butyl, benzyl, phenylcarbonyl or benzylcarbonyl optionally substituted in the phenyl ring by methyl, methoxy, chloro or bromo; furylcarbonyl, thienylcarbonyl, pyrrolylcarbonyl or indolylcarbonyl.

Preferably R is hydrogen, methyl, n-butyl, acetyl, benzyl, benzylcarbonyl, phenylcarbonyl or furylcarbonyl.

Most preferably R is methyl.

In a particular aspect of the invention, $R_5$ and $R_6$ together represent $C_3$-or $C_4$-polymethylene when A represents $>C=X$.

Suitable unsaturated, heterocyclic rings represented by the moiety $R_6$.N.$R_5$ include those having 5- or 6- ring atoms, favourably 6- ring atoms.

Suitable optional substituents for the ring atoms of the unsaturated, heterocyclic ring represented by $R_6$.N.$R_5$, include alkyl, hydroxyl, thiol, oxo or thioxo and also those substituents which jointly provide fused rings, for example a fused benzene ring or a further heterocyclic ring, including any heterocyclic ring mentioned above.

Preferably $R_7$ and $R_8$ are independently selected from the class of hydrogen, alkyl, $C_{3-8}$ cycloalkyl, fluoroalkyl, alkylcarbonyl, alkoxycarbonyl, nitro, cyano or amino, providing that at least one of $R_7$ or $R_8$ is not hydrogen. In particular, $R_7$ and $R_8$ may each independently represent hydrogen, acetyl, nitro, cyano, amino, methyl, ethyl, isopropyl, cyclopentyl, trifluoromethyl or pentafluoroethyl, providing that at least one of $R_7$ or $R_8$ is not hydrogen. Preferably, $R_7$ and $R_8$ may each independently represent hydrogen, nitro, cyano, amino, methyl, ethyl, trifluoromethyl or pentafluoroethyl, providing that at least one of $R_7$ or $R_8$ is not hydrogen.

When one of $R_7$ and $R_8$ represents nitro, cyano or alkylcarbonyl, suitably $C_{1-3}$ alkylcarbonyl, the other may represent amino optionally substituted by one or two $C_{1-6}$ alkyl or by $C_{2-7}$ alkanoyl. In particular, when one of $R_7$ and $R_8$ is nitro, cyano or $C_{1-3}$ alkylcarbonyl, the other is amino, methylamino,dimethyl -amino or acetylamino. For example when one of $R_7$ and $R_8$ is nitro or cyano, the other may represent amino.

When one of $R_7$ and $R_8$ is nitro, cyano or alkylcarbonyl, $R_8$ may represent nitro, cyano or alkylcarbonyl.

When one of $R_7$ or $R_8$ represents hydrogen the other is preferably selected from the class of alkyl, $C_{3-8}$ cycloalkyl, fluoroalkyl, alkylcarbonyl, alkoxycarbonyl, nitro, cyano or amino, and in particular the other is selected from acetyl, nitro, cyano, amino, methyl, ethyl, isopropyl, cyclopentyl, trifluoromethyl or pentafluoroethyl.

Unless mentioned to the contrary, $R_7$ suitably represents hydrogen and $R_8$ represents any of the variables mentioned above in regard to $R_7$ or $R_8$.

Unless mentioned to the contrary, $R_8$ suitably represents hydrogen and $R_7$ represents any of the variables mentioned above in regard to $R_7$ or $R_8$.

In one preferred aspect $R_7$ represents hydrogen and $R_8$ represents nitro, cyano, amino, methyl, ethyl, trifluoromethyl or pentafluoroethyl, for example methyl.

In one preferred aspect $R_8$ represents hydrogen and $R_7$ represents nitro, cyano, amino, methyl, ethyl, trifluoromethyl or pentafluoroethyl.

Suitably, p represents zero. Suitably, p represents 2. Preferably, p represents zero.

When used herein the term "halogen" refers to fluorine, chlorine, bromine and iodine; preferably chlorine.

Suitably alkyl groups, or alkyl groups forming part of other groups such as in the alkoxy group, are $C_{1-12}$ alkyl groups especially $C_{1-6}$ alkyl groups e.g. methyl, ethyl, n-propyl, iso-propyl, n-butyl, isobutyl or tert-butyl groups.

Suitably alkenyl groups are $C_{2-12}$ groups especially $C_{2-6}$ alkenyl groups.

Suitable alkynyl groups are $C_{2-12}$ alkynyl groups especially $C_{2-6}$ alkynyl groups.

Suitable polymethylene groups include $C_3$, $C_4$, $C_5$, $C_6$ and $C_7$ polymethylene groups.

Suitable acyloxy groups include alkylcarbonyloxy groups wherein the alkyl group is as defined above.

When used herein the term "fluoroalkyl" includes alkyl groups as defined above when substituted by one or more fluorine atoms, particular examples being trifluoromethyl and pentafluoroethyl.

Suitable pharmaceutically acceptable salts of the compounds of formula (I) include acid addition salts and salts of carboxy groups.

Examples of pharmaceutically acceptable acid addition salts of the compounds of formula (I) includes acid addition salts of optionally substituted amino groups, such as the hydrochloride and hydrobromide salts. Such a salifiable group may form part of an $R_5$ group. It will also be appreciated that when Y in the compound of formula (I) represents N, then the resulting pyridine moiety may yield acid addition salts, such as the hydrochloride or hydrobromide salts.

Examples of pharmaceutically acceptable salts of carboxy groups include metal salts, such as alkali metal salts, or optionally substituted ammonium salts.

Examples of esters of carboxy groups are pharmaceutically acceptable esters such as $C_{1-6}$ alkyl esters.

Examples of amides of carboxyl groups include pharmaceutically acceptable amides such as amides of formula $-CO.NR^sR^t$ wherein $R^s$ and $R^t$ each independently represent hydrogen or $C_{1-6}$ alkyl.

The compounds of formula (I) may also exist in the form of solvates, preferably hydrates, and the invention extends to such solvates.

The compounds of formula (I), may exist in the form of optical isomers. For example chirality is present in those compounds of formula (I) wherein $R_3$ is hydrogen, hydroxy, alkoxy or acyloxy and $R_4$ is hydrogen. Chirality may also arise in those compounds of formula (I) wherein $R_5$ and $R_6$ together represent a linking group $-A^1-A^2-$, as the said linking group may possess up to 4 chiral carbon atoms. The present invention extends to all optical isomers of the compounds of formula (I) whether in the form of single isomers or of mixtures thereof, such as racemates.

The compounds of formula (I) may also exist in geometrical isomeric forms all of which are encompassed by the present invention, including those wherein the $R_6.N.AR_5$ moiety and $R_3$ are disposed either mutually cis with respect to one another or, preferably, mutually trans with respect to one another.

Particular examples of compounds of formula (I) include the compounds prepared in the Examples hereinafter.

The present invention also provides a process for the preparation of a compound of formula (I) or, where appropriate a pharmaceutically acceptable salt thereof, or a pharmaceutically acceptable solvate thereof, which comprises;

i) treating a compound of formula (II):

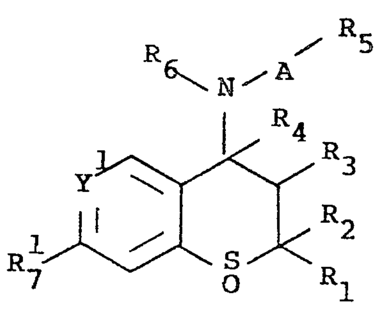

(II)

wherein $R_1$, $R_2$, $R_3$, $R_4$, $R_5$, $R_6$ and A are as defined in relation to formula (I), $R_7{}^1$ represents $R_7$ or a group or atom convertible to $R_7$ and $Y^1$ represents Y or a group convertible to Y,

with an appropriate redox reagent; or

ii) acylating a compound of formula (III):

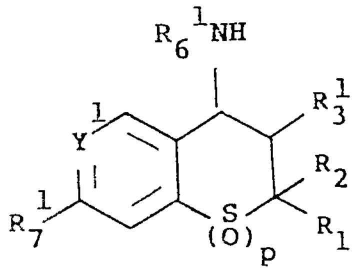

(III)

wherein, $R_1$, $R_2$, $R_7{}^1$ and $Y^1$ are as defined in relation to formula (II), $R_3{}^1$ represents hydroxy, alkoxy or acyloxy, $R_6{}^1$ is hydrogen or alkyl, and p represents zero or an integer 2,

a) with an acylating agent of formula (IV):

$R_{10}$-CO-$L_1$ (IV)

wherein $L_1$ is a leaving group, and $R_{10}$ is hydrogen, substituted or unsubstituted alkyl wherein the substituents are as hereinbefore defined for $R_5$; amino optionally substituted as hereinbefore defined for $R_5$; alkenyl; or optionally substituted aryl or heteroaryl as hereinbefore defined for $R_5$, or a group convertible to $R_5$ as hereinbefore defined, and thereafter, when $R_6$ is hydrogen and $R_{10}$ is $X^1(CH_2)_z$, where z is 3 or 4; and $X^1$ is a leaving group, cyclising the resultant compound;

b) with a compound of formula (V)

$X{=}C{=}N.R_{11}$ (V)

wherein $R_{11}$ is hydrogen, alkyl, alkenyl, alkanoyl optionally substituted by up to three halo atoms, or phenyl optionally substituted by alkyl, alkoxy or halogen; and X is oxygen or sulphur, and thereafter when $R_{11}$ is hydrogen, optionally converting $R_{11}$; or

iii) for compounds of formula (I) wherein $R_5$ and $R_6$ together represent a linking chain of formula $-A^1-A^2$ as defined above in relation to formula (I) by reacting a compound of formula (VI):

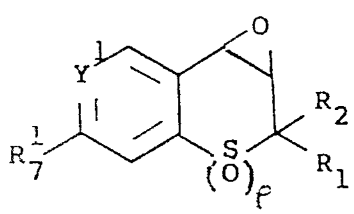

(VI)

wherein $R_1$, $R_2$ and p are as hereinbefore defined in relation to formula (I) and $R_7{}^1$ and $Y^1$ are as defined in relation to formula (III), with a compound of formula (VII):

$R_{13}NHCOR_{12}$ (VII)

wherein $R_{12}$ and $R_{13}$ together represent a linking chain $-A^1-A^2-$; or

iv) for compounds of formula (I) wherein A represents a bond and $R_5$ and $R_6$ together with the nitrogen to which they are attached form the above defined unsaturated heterocyclic ring, by reacting a compound of formula (VI) as defined above with an activated form of a compound of formula (VIIA):

$R_6'NHR_5'$ (VIIA)

wherein $R_5'$ and $R_6'$ form an unsaturated heterocyclic ring as defined above in respect of the variables $R_5$ and $R_6$;

and thereafter if required, carrying out one or more of the following optional steps:

(i) converting a compound of formula (I) into a further compound of formula (I);

(ii) converting $Y^1$ to Y;

(iii) converting $R_7{}^1$ to $R_7$;

(iv) forming a pharmaceutically acceptable salt of the compound of formula (I); or

(v) forming a pharmaceutically acceptable solvate of the compound of formula (I) or a pharmaceutically acceptable salt thereof.

Preferably in (VI) p = 2.

In the process variant i), an appropriate redox reagent for preparing a compound of formula (I) wherein p represents zero is an appropriate deoxygenating agent and an appropriate redox reagent for preparing a compound of formula (I) wherein p represents 2, is an appropriate oxidising agent.

Appropriate deoxygenating agents include reagents such as those disclosed in Tetrahedron, 1988, 44(2),6537 and also trialkylphosphite/iodine such as tri($C_{1-6}$)alkylphosphite/iodine and especially triethylphosphite/iodine, triphenylphosphine/carbon tetrachloride or triphenylphosphine/sodium iodide/iodine; preferably triethylphosphite/iodine.

Appropriate oxidising agents include peracid oxidising agents, such as a perbenzoic acid, in particular 3-chloroperbenzoic acid.

The reaction conditions for the reaction between the compound of formula (II) and the appropriate redox reagent will of course depend upon the nature of the reagent chosen and the nature of the compounds of formulae (I) and (II).

When the appropriate redox reagent is an appropriate deoxygenating agent, such as triethylphosphite/iodine, the reaction may be carried out in any suitable solvent, for example in acetonitrile, at low to medium temperatures, for example at ambient temperature.

When the appropriate redox reagent is an appropriate oxidising agent, for example a peracid such as 3-chloroperbenzoic acid, the reaction may be carried out in any suitable solvent, for example dichloromethane, at low to medium temperatures, for example at ambient temperature.

The compounds of formula (II) are believed to be novel compounds and accordingly they form part of the present invention. The compounds of formula (II) are especially useful as intermediates for the preparation of compounds of formula (I).

In the process variant ii) a) acylation of a compound of formula (III) with an acylating agent of formula (IV), the leaving group $L_1$ is a group that is displaceable by a primary or secondary amino nucleophile. Examples of such a group include $C_{1-4}$ alkanoyloxy, and halogen, such as chloro and bromo. When the leaving group $L_1$ is either of these examples, the acylating agent of formula (IV) is either an acid anhydride or an acid halide. When it is an acid anhydride, it may be a mixed or simple anhydride. If it is a mixed anhydride, it may be prepared in situ from a carboxylic acid and an acid halide, although this is less preferred than using the halide itself. When $L_1$ is hydroxy, conventional coupling methods using dicyclohexylcarbodiimide are suitable.

In process variant ii) a), when $R_5$ in the desired compound of formula (I) is an optionally substituted amino-substituted alkyl group as hereinbefore defined, it is preferred that $R_{10}$ is a group convertible to the $R_5$ substituted alkyl group as hereinbefore defined, in particular that it is $C_{1-6}$ alkyl substituted by halo, especially bromo. The $R_{10}$ halo substituent in the resultant compound of process variant ii) a) may be converted to an $R_5$ substituent which is amino optionally substituted as hereinbefore defined by a conventional amination reaction with ammonia or a corresponding alkyl- or dialkylamine.

Less favourably, $R_{10}$ may be $C_{1-6}$ alkyl substituted by protected amino, protected $C_{1-6}$ alkylamino or amino substituted by two independent $C_{1-6}$ alkyl groups, it being necessary to protect the $R_9$ amino function in process variant ii) a).

When the acylating agent of formula (IV) is an acid anhydride, the acylation of the compound of formula (III) may be carried out in the presence of an acid acceptor, such as sodium acetate, optionally using the anhydride as the solvent.

When the acylating agent of formula (IV) is an acid halide, the acylation of the compound of formula (III) is, preferably, carried out in a non-aqueous medium, such as dichloromethane, in the presence of an acid acceptor, such as triethylamine, trimethylamine, pyridine, picoline or calcium, potassium or sodium carbonate.

When $R_3{}^1$ in a compound of formula (III) is hydroxy, there is a risk of a side-reaction between the hydroxy group and the acylating agent of formula (IV). However, the reaction may be carried out under controlled conditions such that only the amine, $R_6{}^1$NH-is acylated, for example, by using a $C_{2-9}$ acyloxy group as the leaving group $L_1$, in the acylating agent of formula (IV) in the manner as previously described for an acid anhydride, and/or effecting the reaction at relatively low temperature, e.g. at below 10°C.

Alternatively $R_3{}^1$ may be acyloxy, suitably $C_{1-7}$ acyloxy, in a compound of formula (III), although less preferably if $R_3$ in the resultant compound of formula (I) is to be hydroxy, and, after reaction with the acylating agent of formula (IV), be converted into hydroxy, as described hereinafter.

When $R_{10}$ is $X^1(CH_2)_z$ wherein the variables $X^1$ and z are as hereinbefore defined, the leaving group $X^1$ is a group that is displaceable by a secondary amino nucleophile adjacent to a carbonyl function. A preferred example is chloro.

The cyclisation reaction in the reaction wherein $R_{10}$ represents $X^1(CH_2)_z$ as defined above is preferably carried out in an inert solvent such as dimethylformamide.

In process variant ii) b), when $R_{11}$ in a compound of formula (V) is alkyl, alkanoyl optionally substituted as hereinbefore defined, or phenyl optionally substituted as hereinbefore defined, the reaction between the

compounds of formulae (III) and (V) is, preferably, carried out in a solvent, such as methylene chloride, at below room temperature, in particular below 10°C.

When $R_{11}$ is hydrogen, the reaction between the compounds of formulae (III) and (V) is, preferably, carried out using a corresponding alkali metal cyanate or thiocyanate, for example that of sodium or potassium, in an optionally methanolic aqueous medium acidified with a mineral acid, such as dilute hydrochloride acid. A slightly elevated temperature such as 50 to 90°C is apt.

In the process variant iii), that is the reaction between a compound of formula (VI) and a compound of formula (VII), it is particularly preferred that the reaction is carried out under basic conditions so as to facilitate the formation of the anion of the compound of formula (VII), for example, in the presence of an alkali metal base such as potassium t-butoxide or sodium hydride.

The reaction between the compounds of formula (VI) and (VII) may be carried out in any suitable aprotic solvent at a temperature that provides a convenient rate of formation of the compound of formula (I), such as at ambient temperature or at a slightly elevated temperature, for example 40°C.

Conveniently, the compound of formula (VII) may itself be used as the solvent for the reaction between compounds of formulae (VI) and (VII).

In process variant iv), a suitable activated form of a compound of formula (VIIA) is an ionic form. Thus in the reaction between a compound of formula (VI) and a compound of formula (VIIA), it is preferred that the reaction is carried out under basic conditions so as to facilitate the formation of the anion of the compound of formula (VIIA), for example, in the presence of an alkali metal base such as potassium t-butoxide or sodium hydride.

The reaction between the compounds of formulae (VI) and (VIIA) may be carried out in any suitable aprotic solvent, for example dimethylsulphoxide, at a temperature that provides a convenient rate of formation of the compound of formula (I), such as at ambient temperature or at an elevated temperature, but conveniently at ambient temperature.

Suitable conversions of a compound of formula (I) to a further compound of formula (I) include:

(i) converting $R_3$ in the resulting compound of formula (I) into another $R_3$;

(ii) converting a compound of formula (I) wherein $R_3$ and $R_4$ represent hydroxy and hydrogen respectively to give another compound of formula (I), wherein $R_3$ and $R_4$ together represent a bond;

(iii) reducing any compound of formula (I) wherein $R_3$ and $R_4$ together represent a bond; to give another compound of formula (I), wherein $R_3$ and $R_4$ each represent hydrogen;

(iv) thiating the $R_6$.N.CO.$R_5$ group in the compound of formula (I) to give a compound wherein X is sulphur;

(v) converting a compound of formula (I) wherein p represents zero to a compound of formula (I) wherein p represents 2;

(vi) converting a compound of formula (I) wherein p represents 2 to a compound of formula (I) wherein p represents zero; or

(vii) when $R_3$ is other than hydrogen, interconverting the cis and trans mutual configuration of the variables $R_3$ and $R_4$.

The reaction of the compounds of formulae (III) with (IV) or (V) results in a compound of formula (I) wherein $R_3$ is hydroxy, alkoxy or acyloxy, whereas the reaction of the compounds of formulae (VI), (VII) and (VIIA) results in a compound of formula (I) wherein $R_3$ is hydroxy. Examples of an optional conversion of $R_3$ in a compound of formula (I) into another $R_3$ are generally known in the art. For example, when $R_3$ is hydroxy, it may be alkylated using an alkyl iodide in an inert solvent, such as toluene, in the presence of a such as sodium hydride or potassium hydroxide, or it may be acylated using a carboxylic acid chloride or or an appropriate anhydride in a non-hydroxylic solvent, such as toluene or dichloromethane, in the presence of an acid acceptor, such as triethylamine. When $R_3$ is acyloxy or alkoxy, it may be converted into hydroxy by means of any conventional dealkylation method, such as by treatment with trimethylsilyliodide in an aprotic solvent. In addition when $R_3$ is acyloxy it may be converted into hydroxy by conventional hydrolysis using, for example, dilute mineral acid. Preferably the said conversion is carried out when p represents 2.

The optional conversion of a compound of formula (I), wherein $R_3$ and $R_4$ are hydroxy and hydrogen respectively, into another compound of formula (I), wherein $R_3$ and $R_4$ together are a bond, may be carried out by dehydration under conventional dehydration conditions, for example, by using a dehydrating agent, such as sodium hydride, in inert solvent, such as dry tetrahydrofuran, at reflux temperature; alternatively the hydroxy group represented by $R_3$ may be converted into a leaving group such as a mesyloxy or tosyloxy group and the resulting compound treated with a base such as sodium hydride to provide the compound of formula (I) wherein $R_3$ and $R_4$ together represent a bond. Preferably the said conversion is carried out when p represents 2.

The reduction of a compound of formula (I), wherein $R_3$ and $R_4$ together are a bond, into another compound of formula (I), wherein $R_3$ and $R_4$ are each hydrogen, may be carried out by hydrogenation using a catalyst of palladium on charcoal. Preferably the said conversion is carried out when p represents 2.

The thiation of the $R_6$.N.CO.$R_5$ group in a compound of formula (I) to give another compound of formula (I), wherein X is sulphur, is, preferably, carried out with conventional thiation agents, such as hydrogen sulphide, phosphorus pentasulphide and Lawesson's reagent (p-methoxyphenylthiophosphine sulphide dimer). The use of hydrogen sulphide and phosphorus pentasulphide may lead to side-reactions and, therefore, the use of Lawesson's reagent is preferred. Preferably the said conversion is carried out when p represents 2.

The thiation reaction conditions are conventional for the thiation agent employed. For example, the use of hydrogen sulphide is, preferably, acid catalysed by, for example, hydrogen chloride in a polar solvent, such as acetic acid or ethanol. The preferred use of Lawesson's reagent is, preferably, carried out under reflux in a dry solvent, such as toluene or methylene chloride.

The optional conversion of a compound of formula (I) wherein p represents zero into a compound of formula (I) wherein p represents 2 may be carried out by converting the compound of formula (I) into the corresponding compound of formula (II) and thereafter converting the compound of formula (II) into the required compound of formula (I). These interconversions of formulae (I) may be carried out under conditions analogous to those described above to prepare compounds of the formula (I) from compounds of formula (II).

The conversion of a compound of formula (I) wherein p represents 2 into a compound of formula (I) wherein p represents zero, is suitably carried out directly using the appropriate procedure.

The interconversion of the cis and trans configuration of the variables $R_3$ and $R_4$ is generally carried out by changing the configuration of variable $R_3$, especially when $R_3$ represents hydroxyl, by means of any convenient conventional procedure. Preferably the said conversion is carried out when p represents 2.

The conversion of a compound of formula (I) into another compound of formula (I) wherein p represents zero is suitably carried out by first converting the said compound of formula (I) into the corresponding compound of formula (II), carrying out the appropriate conversion and then converting the compound of formula (II) into the corresponding compound of formula (I). The nature of the appropriate conversions of the compounds of formula (II) and the appropriate procedures are analagous to those mentioned above for the compounds of formula (I). It will be appreciated from the foregoing that it is preferred that compounds of formula (I), wherein p represents zero are prepared directly from the corresponding compound of formula (II).

Any conversion of $Y^1$ to Y or $R_7{}^1$ to $R_7$ may be carried out using the appropriate conventional chemical procedure.

The optional formation of a pharmaceutically acceptable salt, when the resulting compound of formula (I) contains a salifiable group, may be carried out conventionally. Similarly, pharmaceutically acceptable solvates, for example hydrates, may be prepared using any convenient conventional procedure.

A compound of formula (II) may be prepared either:

i) by reacting a compound of formula (IIIA):

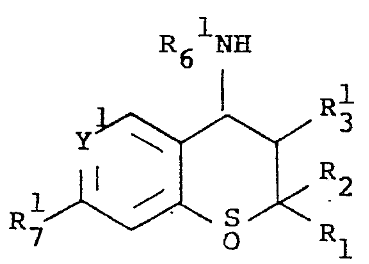

(IIIA)

wherein $R_1$, $R_2$, $R_3{}^1$, $R_6{}^1$, $R_7{}^1$ and $Y^1$ are as defined in relation to formula (III), with either an acylating agent of the hereinbefore defined formula (IV), or with a compound of the hereinbefore defined formula (V); or

ii) by reacting a compound of formula (VIA):

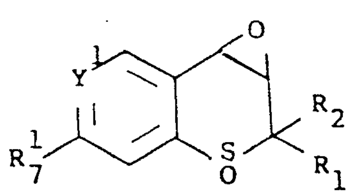

(VIA)

wherein $R_1$, $R_2$, $R_7{}^1$ and $Y^1$ are as defined in relation to formula (VI), with a compound of the hereinbefore defined formula (VII), and thereafter if required optionally converting $Y^1$ to Y or $R_7{}^1$ to $R_7$.

The reaction of the compound of formula (IIIA) with either the compound of formula (IV) or the compound of formula (V), may suitably be carried out under analogous conditions to those described above for the reaction of the compound of formula (III) with either the compound of formula (IV) or the compound of formula (V). The reaction between the compounds of formulae (VIA) and (VII) may suitably be carried out under analogous conditions to those described above for the reaction between the compounds of formulae (VI) and (VII).

A compound of formula (III) may be prepared by reacting a compound of formula (VI), as defined

hereinbefore, with a compound of formula (VIII):

$R_6{}^1NH_2$ (VIII)

wherein $R_6{}^1$ is as defined hereinbefore; and optionally converting $R_3{}^1$ hydroxyl in the resulting compound of formula (III) into another $R_3{}^1$.

The reaction between the compounds of formula (VI) and (VIII) is normally carried out in a solvent, such as a $C_{1-4}$ alcohol, in particular methanol, ethanol or propanol at an ambient or an elevated temperature, for example 12 to 100°C. The reaction proceeds particularly smoothly if carried out in ethanol under reflux.

A compound of formula (IIIA) may be prepared by reaction between the compounds of the hereinbefore defined formulae (VIA) and (VIII). Suitable reaction conditions for the reaction between compounds of formulae (VIA) and (VIII) may be those described above in relation to the reaction between compounds of formulae (VI) and (VIII).

The resulting compound of formula (III) or (IIIA) may be recovered from the reaction mixture by removal of the solvent, for example, by evaporation under reduced pressure. Any epoxide impurity may be removed conventionally, for example by chromatography.

The optional conversion of the hydroxy group for $R_3{}^1$ in the resulting compound of formula (III) into a alkoxy or acyloxy group, or any analogous conversion carried out in the preparation of compounds of formula (IIIA), may be carried out as described hereinbefore in relation to the corresponding conversion of $R_3$ in a compound of formula (I).

A compound of formula (VI) or (VIA) may be prepared by reaction of a compound of formula (IX):

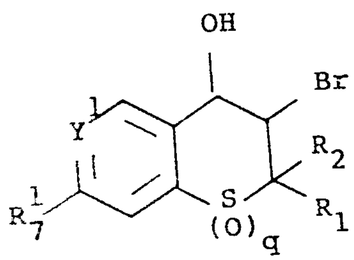

(IX)

wherein $R_1$, $R_2$, $R_7{}^1$ and $Y^1$ are as hereinbefore defined and q represents zero, 1 or 2, providing that q represents zero or an integer 2 for preparing compounds of formula (VI) and g represents an integer 1 for preparing compounds of formula (VIA), and wherein the bromine atom is trans to the hydroxy group; with a base, such as potassium hydroxide, in a solvent, such as diethyl ether or aqueous dioxan.

A compound of formula (IX) may be prepared by reaction of a compound of formula (X):

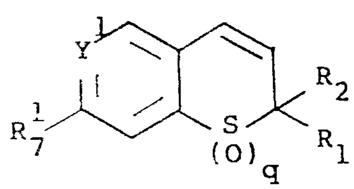

(X)

wherein $R_1$, $R_2$, $R_7{}^1$, $Y^1$ and g are as defined in relation to formula (IX), with N-bromosuccinimide in a solvent, such as aqueous dimethyl sulphoxide or acetone, preferably acetone; and thereafter if required converting a compound of formula (IX) into another compound of formula (IX).

A compound of formula (X) may be prepared by oxidising a compound of formula (XI):

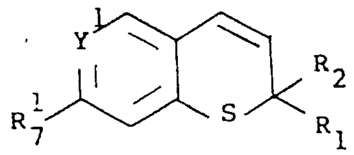

(XI)

wherein $R_1$, $R_2$, $R_7{}^1$ and $Y^1$ are as hereinbefore defined, with an appropriate oxidising agent; and thereafter if required converting a compound of formula (X) into a further compound of formula (X).

An appropriate oxidising agent for oxidising a compound of the above defined formula (XI) into a compound of formula (X) is a peracid oxidising agent, for example 3-chloroperbenzoic acid, or a periodate oxidising agent, for example sodium metaperiodate.

The oxidation of the compounds of formula (XI) may be carried out under any suitable conditions appropriate to the precise nature of the compound of formula (XI); thus when a periodate oxidising agent is used, for example sodium metaperiodate, the reaction is conveniently carried out in methanol at low to ambient temperature, suitably at ambient temperature.

A compound of formula (VI), wherein p represents 2, or a compound of formula (VIA), may also be prepared by reacting a compound of the hereinbefore defined formula (XI) with a peracid, preferably a perbenzoic acid, and in particular with 3-chloroperbenzoic acid.

The reaction between the compound of formula (XI) and the peracid, such as 3-chloroperbenzoic acid, may be carried out in any suitable inert solvent such as dichloromethane at any suitable temperature, conveniently at ambient temperature.

It will be appreciated that the preparation of the compounds of formula (VI) by oxidation of compounds of formula (XI) will proceed via the corresponding compound of formula (VIA). The compounds of formula (VIA) are therefore intermediates to the corresponding compounds of formula (VI). In general it is more convenient to allow the oxidation of the compounds of formula (XI) to proceed to the compounds of formula (VI) without isolating the corresponding compounds of formula (VIA) although the compounds of formula (VIA) may be prepared by this method by utilising conventional synthetic techniques, such as chromatography to monitor the oxidation and subsequently to isolate the compounds of formula (VIA) at the appropriate stage of the reaction.

The optional conversion of a compound of formula (III) into another compound of formula (III), the optional conversion of a compound of formula (VI) to (VIA) or vice versa, the optional conversion of a compound of formula (IX) into another compound of formula (IX) or the optional conversion of a compound of formula (X) into a further compound of formula (X) suitably includes a conversion wherein the oxidation state of the S-atom in any of the abovementioned compounds is converted from one of the above described values into another of the above described values for example, where applicable, the conversions S to SO, S to $SO_2$ and $SO_2$ to S and especially the conversions S to SO and SO to $SO_2$.

Suitable conditions for effecting the last above-mentioned conversions are those used conventionally in the art, for example the conversions may be carried out in an analogous fashion to the preparation of a compound of formula (I) from a compound of formula (II).

As mentioned previously, some of the compounds of formula (I) may exist in optically active forms, and the processes of the present invention produce mixtures of such forms. The individual enantiomers may be resolved by conventional methods.

It is preferred that the compounds of formula (I) are isolated in substantially pure form.

The intermediates of formulae (III), (VI), (IX) or (X) are believed to be novel and accordingly they form part of the present invention.

The intermediates of formulae (IV), (V), (VII), (VIIA) or (VIII) are known and may be prepared using conventional procedures, for example those disclosed in Advanced Organic Chemistry, 3rd Edition, (1985), Published by John Wiley and Sons.

The compounds of formula (XI) are either known compounds or they may be prepared using methods analogous to those used to prepare known compounds, for example those disclosed in Synthesis 1987, 149.

The compounds of formula (I), the pharmaceutically acceptable salts thereof or the pharmaceutically acceptable solvates thereof, have been found to have bronchodilator activity and/or blood-pressure lowering activity. They are therefore useful in the treatment of respiratory tract disorders, such as reversible airways obstruction, diverticular disease and asthma and also hypertension. They may also be of potential use in the treatment of other disorders hereinbefore described.

The present invention accordingly provides a pharmaceutical composition which comprises a compound of formula (I), or a pharmaceutically acceptable salt thereof or a pharmaceutically acceptable solvate thereof, and a pharmaceutically acceptable carrier.

The compositions are preferably adapted for oral administration. However, they may be adapted for other modes of administration, for example in the form of a spray, aerosol or other conventional method for inhalation, for treating respiratory tract disorders; or parenteral administration for patients suffering from heart

failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium. phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

The compositions may contain from 0.1% to 99% by weight, preferably from 10-60% by weight, of the active material, depending on the method of administration.

The present invention further provides a method of treatment of respiratory tract disorders or hypertension in mammals including man, which method comprises administering an effective, non-toxic amount of a compound of formula (I), or a pharmaceutically acceptable salt thereof or a pharmaceutically acceptable solvate thereof to the mammal in need thereof.

The present invention also provides a method of treatment of disorders associated with smooth muscle contraction of the gastro-intestinal tract and the abovementioned cardiovascular disorders in mammals including man, which method comprises administering an effective, non-toxic amount of a compound of formula (I), or a pharmaceutically acceptable salt thereof or a pharmaceutically acceptable solvate thereof to the mammal in need thereof.

An effective amount wil depend on the relative efficacy of the compounds of the present invention, the severity of the respiratory tract disorder or hypertension being treated and the weight of the sufferer. However, a unit dose form of a composition of the invention may contain from 0.01 to 100mg of a compound of the invention (0.01 to 10mg via inhalation) and more usually from 0.1 to 50mg, for example 0.5 to 25mg such as 1, 2, 5, 10, 15 or 20 mg. Such compositions may be administered from 1 to 6 times a day, more usually from 2 to 4 times a day, in a manner such that the daily dose is from 0.02 to 200mg for a 70 kg human adult and more particularly from 0.05to 100mg.

Suitable dosages for the treatment of disorders associated with smooth muscle contraction of the gastro intestinal tract and the abovementioned cardiovascular disorders are those described for the treatment of respiratory tract disorders.

The present invention further provides a compound of formula (I) or a pharmaceutically acceptable salt thereof, or a pharmaceutically acceptable solvate thereof, for use in the treatment of respiratory tract disorders or hypertension or disorders associated with smooth muscle contraction of the gastro intestinal

tract or the abovementioned cardiovascular disorders.

The present invention further provides the use of a compound of formula (I), or a pharmaceutically acceptable salt thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders.

The following Procedures relate to the preparation of intermediates and the following Examples relate to the preparation of compounds of formula (I).

Example 1

Trans 3,4-Dihydro-4-(2-oxopiperidin-1-yl)-2,2,6-trimethyl-2H-1-benzothiapyran-3-ol

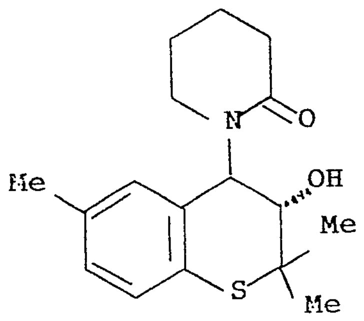

Iodine ( 0.126g, 0.496mmol) in dry acetonitrile (5ml) was added dropwise to a stirred solution of triethylphosphite (0.079g, 0.476mmol) and 3,4-dihydro-4β-(2-oxopiperidin-1-yl)-2,2,6-trimethyl-2H-1-benzothiapyran-3α-ol-1α-oxide (0.157g, 0.489mmol) in dry acetonitrile (10ml) at ambient temperature until a faint brown colour persisted. The solution was allowed to stir for 3.5h after which the solvent was evaporated under reduced pressure and the residue partitioned between dichloromethane and sodium thiosulphate solution. The aqueous phase was further extracted and the combined organic layers were dried ($MgSO_4$), filtered and evaporated to give an oil which was chromatographed on alumina. Further chromatography gave an oil (0.092g) which was triturated with ether and filtered to give trans 3,4-Dihydro-4-(2-oxopiperidin-1-yl)-2,2,6-trimethyl-2H-1-benzothiapyran-3-ol as a white solid (0.035g, 23%), m.p. 147-155°C.

$^1H$ n.m.r. ($CDCl_3$):

δ 1.37 (3H, s); 1.41 (3H, s); 1.68-1.78 (2H, m); 1.80-1.90 (2H, m); 1.5-2.25 (1H, broad, disappears with $D_2O$); 2.27 (3H, s); 2.56-2.62 (2H, m); 2.85-2.92 (1H, m); 3.02-3.11 (1H, m); 4.10 (1H, d, J = 9 Hz); 5.92 (1H, d, J = 9Hz); 6.86 (1H, s); 6.95 (1H, d, J = 8 Hz); 7.02 (1H, d, J = 8 Hz):

Mass spectrum

Found $M^+$ 305.1451. $C_{17}H_{23}NO_2S$ requires 305.1450

Example 2

4-(2-Oxopyrrolidin-1-yl)-2,2,6-trimethyl-2H-1-benzothiapyran-1,1-dioxide

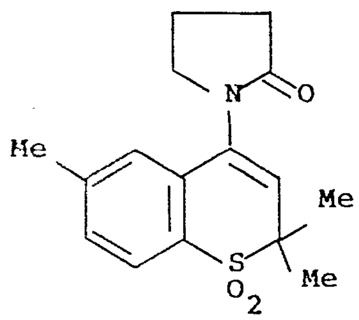

Sodium hydride (0.129g, 3.25mmol of a 60% dispersion in oil) was added to a stirred solution of 3,4-dihydro-3,4-epoxy-2,2,6-trimethyl-2H-1-benzothiapyran-1,1-dioxide (0.412g, 1.73mmol) and 2-pyrrolidinone (0.225g, 2.65mmol) in dry dimethyl sulphoxide at ambient temperature under nitrogen. The solution was left to stir for 48h. The reaction mixture was poured into water, extracted with ethyl acetate (3 x 50ml), the combined organic layers washed successively with water (3 x 50ml) and brine, dried, filtered and evaporated to an oil which was chromatographed on silica. Elution with chloroform gave an oil (0.317g, 60%) which was triturated with ether and filtered to give 4-(2-oxopyrrolidin-1-yl)-2,2,6-trimethyl-2H-1-benzothiapyran-1,1-dioxide, m.p. 135-8°C.

$^1$H n.m.r. ($CDCl_3$):
δ 1.55 (6H, s); 2.23 (2H, m); 2.44 (3H, s); 2.60 (2H, t); 3.59 (2H, t); 6.03 (1H, s), 6.96 (1H, s); 7.34 (1H, dd., J = 8 Hz, 1 Hz); 7.94 (1H, d, J = 8 Hz).

Anal:
Found C, 62.88; H, 6.28; N, 4.56%.
Requires C 62.92; H, 6.27; N, 4.59% for $C_{16}H_{19}NO_3S$

Example 3

Trans 3,4-dihydro-4-(2-oxopiperidin-1-yl)-2,2,6-trimethyl-2H-1-benzothiapyran-3-ol-1,1-dioxide

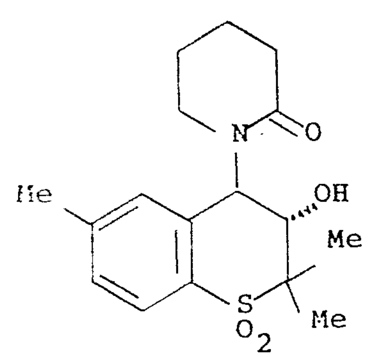

3-Chloroperbenzoic acid (0.11g, 0.508mmol of assumed 80% pure material) in dichloromethane (10ml) was added to a stirred solution of 3,4-dihydro-4β -(2-oxopiperidin-1-yl)-2,2,6-trimethyl-2H-1-benzothiapyran-3α-ol-1α-oxide (0.153g, 0.477mmol) in dichloromethane (10ml) at ambient temperature. The solution was allowed to stir for 2.75h, poured into sodium hydroxide solution (2M, 25ml), the phases separated and the aqueous phase further extracted. The organic layers were combined, dried ($MgSO_4$), filtered and evaporated to a foam which was chromatographed on silica. Elution with chloroform gave an oil (0.144g) which, after trituration with ether and filtration, gave trans 3,4-dihydro-4-(2-oxopiperidin-1-yl)-2,2,6-trimethyl-2H-1-benzothiapyran-3-ol-1,1-dioxide as a white solid (0.095g, 59%), m.p. 224-228°C.

$^1H$ n.m.r. ($CDCl_3$):
δ 1.33 (3H, s); 1.60 (3H, s); 1.72-1.90 (4H, m); 2.41 (3H, s); 2.59-2.64 (2H, m); 2.87-2.94 (1H, m); 3.14-3.21 (1H, m); 3.25-3.75 (1H, broad, disappears with $D_2O$); 4.47 (1H, d, J = 9 Hz); 6.00 (1H, d, J = 9 Hz); 7.01 (1H, s), 7.31 (1H, dd, J = 8 Hz, 0.6 Hz); 7.87 (1H, d, J = 8 Hz).

Anal:
Found C, 60.57; H, 7.16; N, 4.01%.
Requires C, 60.49; H, 6.87; N, 4.15% for $C_{17}H_{23}NO_4S$.

Procedure 1

2,2,6-Trimethyl-2H-1-benzothiapyran-1-oxide

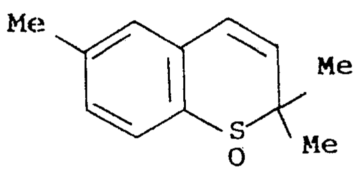

Sodium metaperiodate (3.94g, 0.018mol) in water (30ml -warmed to effect solution) was added dropwise to a stirred solution of 2,2,6-trimethyl-2H-1-benzothiapyran (3.03g, 0.016mol) in methanol (120ml) and, after complete addition of oxidant, the solution was heated under reflux for 1h. The methanol was evaporated under reduced pressure and the aqueous layer was extracted with chloroform (3 x 50ml). The organic layers were combined, washed with brine (2 x 50ml), dried ($MgSO_4$), filtered and evaporated under reduced pressure to give an oil (3.64g) which was chromatographed on silica. Elution with light petroleum (bp 40-60°C): ether (1:3) gave 2,2,6-trimethyl-2H-1-benzothiapyran-1-oxide (3.085g, 94%) as an oil.

$^1H$ n.m.r. ($CDCl_3$):
δ 1.28 (3H, s); 1.51 (3H, s); 2.38 (3H, s); 5.78 (1H, d, J = 10 Hz); 6.6 (1H, d, J = 10 Hz); 7.09 (1H, s); 7.23 (1H, d, J = 8 Hz); 7.67 (1H, d, J = 8 Hz).

Procedure 2

3β-Bromo-3,4-dihydro-2,2,6-trimethyl-2H-1-benzothiapyran-4α-ol-1α-oxide

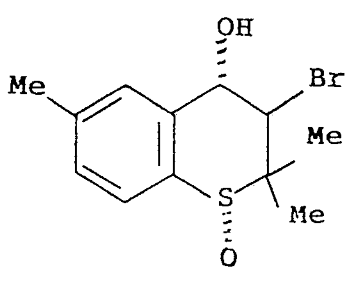

N-Bromoacetamide (3.18g, 0.023mol) was added portionwise to a stirred solution of 2,2,6-trimethyl-2H-1-benzothiapyran-1-oxide (2.198g, 0.011mol) in acetone (107ml) containing water (20ml) at ambient temperature. The solution was stirred for 4.5h, the acetone evaporated under reduced pressure and the residue partitioned between chloroform and water. The organic layer was washed with sodium thiosulphate solution (x2), dried ($MgSO_4$) filtered and evaporated to give a solid (2.49g) which was triturated with ether and filtered to give 3β-bromo-3,4-dihydro-2,2,6-trimethyl-2H-1-benzothiapyran-4α-ol-1α-oxide, 2.02g (63%). Chromatography on silica and elution with chloroform gave material with m.p. 173-177°C.

$^1H$ n.m.r. (DMSO-$d_6$):
δ 1.11 (3H, s); 1.53 (3H, s); 2.40 (3H, s); 4.78 (1H, dd, J = 9.5 Hz, 8 Hz); 4.85 (1H, d, J = 9.5 Hz); 6.44 (OH, d, J = 8 Hz); 7.33 (1H, d, J = 7.6 Hz); 7.57 (1H, s); 7.61 (1H, d, J = 7.6 Hz).

Anal:
Found C, 47.48; H, 5.09%.
Requires C, 47.53; H, 4.99% for $C_{12}H_{15}BrO_2S$.

Procedure 3

Cis 3,4-dihydro-3,4-epoxy-2,2,6-trimethyl-2H-1-benzothiapyran-1-oxide

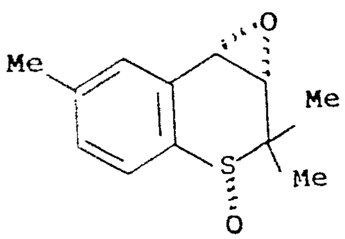

Powdered potassium hydroxide (3.9g, 70mmol) was added in one portion to a stirred solution of 3β-bromo-3,4-dihydro-2,2,6-trimethyl-2H-1-benzothiapyran-4α-ol-1α-oxide (1.974g, 6.5mmol) in dry tetrahydrofuran (100ml) at ambient temperature. The reaction mixture was stirred for 1h, filtered through celite and the filtrate evaporated to give cis 3,4-dihydro-3,4-epoxy-2,2,6-trimethyl-2H-1-benzothiapyran-1-oxide as a white solid (1.46g, 100%), m.p. 126-139° C, which was used without further purification.

$^1H$ n.m.r. ($CDCl_3$):
δ 1.17 (3H, s); 1.67 (3H, s); 2.44 (3H, s); 3.63 (1H, d, J = 3.8 Hz); 3.97 (1H, d, J = 3.8 Hz); 7.35 (1H, d, J = 7.7 Hz); 7.44 (1H, s), 7.66 (1H, d, J = 7.7 Hz).

Procedure 4

3,4-Dihydro-4β-(2-oxopiperidin-1-yl)-2,2,6-trimethyl-2H-1-benzothiapyran-3α-ol-1α-oxide

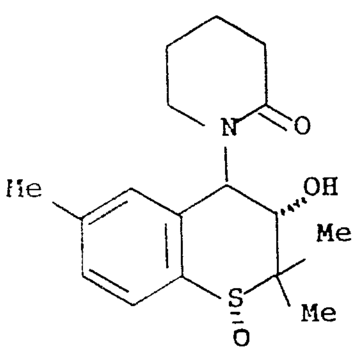

Sodium hydride (0.147g, 3.7mmol of a 60% dispersion in oil) was added in one portion to a stirred solution of cis 3,4-dihydro-3,4-epoxy-2,2,6-trimethyl-2H-1-benzothiapyran-1-oxide (0.665g, 3 mmol) in 2-piperidone (6ml) at 40° C under nitrogen. The reaction mixture was stirred for 2.25h and then a further portion of sodium hydride (0.028g) was added. Stirring was continued for another 1.75h and then the reaction mixture was poured into water and extracted with chloroform (3 x 50ml). The combined organic layers were washed with water (x7) until

no piperidone was present, dried ($MgSO_4$), filtered and evaporated to give an oil which was chromatographed on silica. Elution with chloroform gave starting material (0.073g) followed by the 3,4-ene of the title compound. Elution with methanol-chloroform (5-7.5: 95-92.5) gave an oil (0.661g) which was triturated with ether and filtered to give 3,4-dihydro-4β-(2-oxopiperidin-1-yl)-2,2,6-trimethyl-2H-1-benzothiapyran-3α-ol-1α-oxide as a white solid (0.437g, 46%), m.p. 193-198°C.

$^1$H n.m.r. ($CDCl_3$):
δ 1.14 (3H, s); 1.64 (3H, s); 1.69-1.91 (4H, m); 2.39 (3H, s); 2.56-2.62 (2H, m); 2.88-3.04 (2H, m); 3.94 1H, d, J = 9 Hz); 5.97 (1H, bd, J = 9 Hz); 6.97 (1H, s), 7.32 (1H, d, J = 8 Hz); 7.69 (1H, d, J = 8 Hz), (no OH proton seen but HOD resonance appears after $D_2O$ wash).

Anal:
Found C, 63.53; H, 7.21; N, 4.36% for $C_{17}H_{23}NO_3S$.

Procedure 5

3,4-Dihydro-3,4-epoxy-2,2,6-trimethyl-2H-1-benzothiapyran-1,1-dioxide

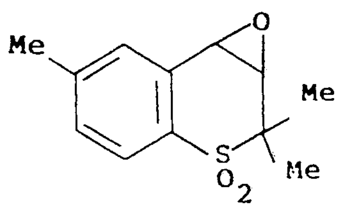

3-Chloroperbenzoic acid (13.2g, 0.061mol of assumed 80% pure material) in 1,2-dichloroethane (120ml) was added dropwise to a stirred solution of 2,2,6-trimethyl-2H-1-benzothiapyran 2.92g, 0.015mol) and 2,6-di-t-butyl-4-cresol (0.050g) in 1,2-dichloroethane (25ml) at ambient temperature. After 0.5h at ambient temperature the reaction mixture was heated under reflux for 4h. The reaction was then cooled, filtered, the filtrate evaporated and the residue chromatographed on alumina. Elution with chloroform gave an oil which was rechromatographed on silica. Elution with chloroform gave 3,4-dihydro-3,4-epoxy-2,2,6-trimethyl-2H-1-benzothiapyran-1,1-dioxide as a white solid (2.33g, 64%), m.p. 148-151°C (sublimes from 130°C).

$^1$H n.m.r. ($CDCl_3$):
δ 1.41 (3H, s); 1.7 (3H, s); 2.45 (3H, s); 3.7 (1H, d, J = 4 Hz); 4.0 (1H, d, J = 4 Hz); 7.42 (1H, d, J = 8 Hz); 7.5 (1H, s), 7.97 (1H, d, J = 8 Hz).

Procedure 6

4-(2-Oxopyrrolidin-1-yl)-2,2,6-trimethyl-2H-1-benzothiapyran-1-oxide

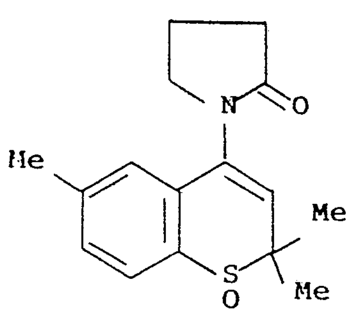

Sodium hydride (0.112g, 2.82mmol of a 60% dispersion in oil) was added to a stirred solution of 3,4-dihydro-3,4-epoxy-2,2,6-trimethyl-2H-1-benzothiapyran-1-oxide (0.308g, 1.39mmol) and 2-pyrrolidone

(0.185g, 2.18mmol) in dry dimethyl sulphoxide (3ml) at ambient temperature under nitrogen. The solution was left to stir for 3 days. The reaction mixture was poured into water, extracted with chloroform and ethyl acetate and the combined organics washed with water, dried ($MgSO_4$), filtered and evaporated to give an oil (0.337g) which was chromatographed on silica. Elution with chloroform gave an oil which was triturated with ether to give 4-(2-oxopyrrolidin-1-yl)-2,2,6-trimethyl-2H-1-benzothiapyran-1-oxide as a white solid, m.p. 139-141°C.

$^1H$ n.m.r. ($CDCl_3$):
δ 1.38 (3H, s); 1.46 (3H, s); 2.20 (2H, m); 2.39 (3H, s); 2.60 (2H, t); 3.53-3.67 (2H, m); 5.79 (1H, s); 6.92 1H, s); 7.28 (1H, d, J = 7.6 Hz); 7.65 (1H, d, J = 7.6 Hz).

Mass Spectrum
Found: $M^+$ 289.1127. $C_{16}H_{19}NO_2S$
Requires 289.1137.

Pharmacological Data

1. Bronchodilator Activity

(a) Bronchodilation in vitro; guinea pig tracheal spiral preparations.

Male guinea pigs (300-600g) were stunned by a blow to the head and bled from the carotid artery. The trachea was exposed, dissected free of connective tissue, and transferred to oxygenated krebs solution at 37°C. Next, spirals (2 per trachea) were prepared by cutting the whole trachea spirally along its longitudinal axis and then dividing this spiral lengthwise. Each preparation was mounted, using silk thread, in a 10ml organ bath filled with krebs solution at 37°C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed cumulatively with the test compound ($10^{-8}$-$2x10^{-5}$M), and finally a maximum relaxation achieved by addition of $10^{-3}$M isoprenaline. The fall in tension evoked by the test compound was expressed as a percentage of the total relaxation evoked in the presence of $10^{-3}$ isoprenaline.

Appropriate concentration-relaxation curves were then constructed and values for potency ($IC_{50}$) were obtained.

The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.

Results

| Compound of Example No: | In vitro $IC_{50}$ value (M) |
|---|---|
| 1 | $2.1 \times 10^{-6}$ |
| 2 | $3.33 \times 10^{-5}$ |
| 3 | $4.9 \times 10^{-5}$ |

2. Antihypertensive Activity

Blood Pressure Lowering Activity

Systolic blood pressures were recorded by a modification of the tail cuff method described by I.M. Claxton, M.G. Palfreyman, R.H. Poyser, R.L. Whiting, European Journal of Pharmacology, 37, 179 (1976). A W+W BP recorder, model 8005 was used to display pulses. Prior to all measurements rats were placed in a heated environment (33.5 ± 0.5°C) before transfer to a restraining cage. Each determination of blood pressure was the mean of at least 6 readings. Spontaneously hypertensive rats (ages 12-18 weeks) with systolic blood pressures >180 mmHg were considered hypertensive.

Results

| Compound of: | dose in mg/kg | Time, post dose in hours | % Change in Systolic Blood Pressure |
|---|---|---|---|
| Example 1 | 1 | 1 | -22 ± 5 (n=6) |
| | | 2 | -17 ± 6 (n=6) |
| | | 4 | -12 ± 11 (n=6) |
| Compound of: | | | |
| Example 2 | 10 | 1 | -50 ± 2 (n=5) |
| | | 2 | -34 ± 4 (n=5) |
| | | 4 | -22 ± 3 (n=4) |
| | | 6 | -15 ± 2 (n=4) |
| Compound of: | | | |
| Example 3 | 30 | 1 | -25 (n=2) |
| | | 2 | -31 ± 7 (n=3) |
| | | 4 | -16 ± 2 (n=3) |
| | | 6 | -14 ± 3 (n=3) |

**Claims**

1. A compound of formula (I):

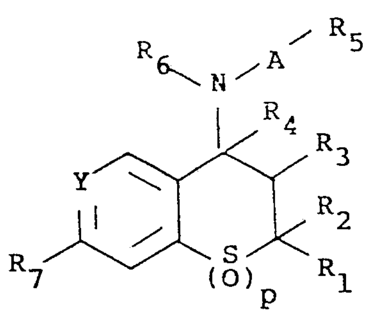

(I)

or, where appropriate, a pharmaceutically acceptable salt thereof, or a pharmaceutically acceptable solvate thereof,characterised in that:
A represents >C=X, wherein
X represents oxygen or sulphur, or A represents a bond;

Y represents N or $N^{+}$-$O^{-}$ or a moiety $CR_8$ wherein
$R_8$ is as defined below;

$R_1$ and $R_2$ independently represent hydrogen or alkyl; or
$R_1$ and $R_2$ together represent a $C_{2-7}$ polymethylene moiety;
$R_3$ represents hydrogen, hydroxy, alkoxy or acyloxy and
$R_4$ is hydrogen or $R_3$ and $R_4$ together represent a bond;
when A represents $>C=X$, then $R_5$ is hydrogen;
alkyl optionally substituted by one or more groups or atoms selected from halogen, hydroxy, alkoxy, alkoxycarbonyl, carboxy or an ester or amide thereof, amino, monoalkylamino or dialkylamino; alkenyl; amino optionally substituted by an alkyl or alkenyl group or by an alkanoyl group optionally substituted by up to three halo atoms, by a phenyl group optionally substituted by alkyl, alkoxy or halogen; substituted or unsubstituted aryl or substituted or unsubstituted heteroaryl; and
$R_6$ represents hydrogen or alkyl;
or $R_5$ and $R_6$ together represent a linking chain of formula $-A^1-A^2-$, $A^1$ being attached to the nitrogen atom of the moiety -N-A- and $A^2$ being attached to the group A on the said moiety, and wherein $A^1$ represents a substituted or unsubstituted methylene group, $A^2$ represents 2 or 3 linking members, one of the linking members optionally representing O, S or NR and the other linking members each independently representing a substituted or unsubstituted methylene group; R represents hydrogen, alkyl, alkanoyl, phenyl $C_{1-4}$-alkyl, arylcarbonyl wherein the aryl group may be substituted or unsubstituted; or R is mono- or bi-cyclic- heteroarylcarbonyl; when A represents a bond, then $R_5$ and $R_6$ together with the nitrogen atom to which they are attached, form an unsaturated heterocyclic ring having 5 to 7 ring atoms, which ring atoms comprise up to 2 further nitrogen atoms and a carbon atom, the carbon atom being substituted with either an oxo group or a thioxo group the remaining ring atoms being substituted or unsubstituted; $R_7$ and $R_8$ are each independently selected from the class of hydrogen, substituted or unsubstituted alkyl, alkoxy, $C_{3-8}$ cycloalkyl, hydroxy, nitro, cyano, halo, formyl, carboxy, a group of formula $R^aT^1$-, $R^bR^cNT$-, $R^aT^2NH$-, $R^dCO.O$-, $R^dCS.O$-, $R^d(OH)CH$-, $R^d(SH)CH$-, $R^dC(=N.OH)$-, $R^dC(N.NH_2)$- or alkenyl optionally substituted by alkylcarbonyl, nitro or cyano, providing that when Y is $CR_8$ then at least one of $R_7$ or $R_8$ is not hydrogen;
$R^a$ represents $R^d$ or $R^dO$- and $R^d$ represents substituted or unsubstituted alkyl, substituted or unsubstituted aryl or substituted or unsubstituted heteroaryl, optional substituents for $R^d$ being up to 3 substituents selected from alkyl, alkoxy, halo, haloalkyl, nitro and cyano;
$R^b$ and $R^c$ each independently represent hydrogen, alkyl or alkylcarbonyl;
T represents a bond or $T^1$, $T^1$ represents -CS- or $T^2$ and $T^2$ represents -CO-, -SO- or $-SO_2$-; and
p represents zero or an integer 2.

2. A compound according to claim 1,
wherein:
X represents oxygen or sulphur;

Y represents N or $N^+-O^-$ or a moiety $CR_8$ wherein $R_8$ is as defined below;
$R^1$ and $R^2$ independently represent hydrogen or $C_{1-6}$ alkyl; or $R_1$ and $R_2$ together represent a $C_{2-7}$ polymethylene moiety;
$R_3$ represents hydrogen, hydroxy, $C_{1-6}$ alkoxy or $C_{1-7}$ acyloxy and $R_4$ is hydrogen or $R_3$ and $R_4$ together represent a bond;
$R_5$ is hydrogen; $C_{1-6}$ alkyl optionally substituted by halogen, hydroxy, $C_{1-6}$ alkoxy, $C_{1-6}$ alkoxycarbonyl, carboxy or amino optionally substituted by one or two independent $C_{1-6}$ alkyl groups; or $C_{2-6}$ alkenyl; amino optionally substituted by a $C_{1-6}$ alkyl or $C_{2-6}$ alkenyl group or by a $C_{1-6}$ alkanoyl group optionally substituted by up to three halo atoms, by a phenyl group optionally substituted by $C_{1-6}$ alkyl, $C_{1-6}$ alkoxy or halogen; or aryl or heteroaryl, either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and $R_6$ represents hydrogen or $C_{1-6}$ alkyl;
or $R_5$ and $R_6$ together represent $-CH_2-(CH_2)_n-Z-(CH_2)_m$-wherein m and n are O to 2 such that $m + n$ is 1 or 2 and Z is $CH_2$, O, S or NR wherein R is hydrogen, $C_{1-9}$ alkyl, $C_{2-7}$ alkanoyl, phenyl $C_{1-4}$- alkyl, naphthylcarbonyl, phenylcarbonyl or benzylcarbonyl optionally substituted in the phenyl or naphthyl ring by one or two of $C_{1-6}$ alkyl, $C_{1-6}$ alkoxy or halogen; or R is mono- or bi-cyclic- heteroarylcarbonyl; when Y is N or $N^+-O^-$, $R_7$ is hydrogen or, when Y is $CR_8$, either one of $R_7$ and $R_8$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkyl-thiocarbonyloxy, $C_{1-6}$ alkyl-thiomethyl, formyl or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino $C_{1-6}$ alkoxysulphinylamino or $C_{1-6}$ alkoxysulphonylamino or ethenyl terminally substituted by $C_{1-6}$ alkylcarbonyl, nitro or cyano, or $-C(C_{1-6}$ alkyl$)NOH$ or $-C(C_{1-6}$ alkyl$)NNH_2$; or one of $R_7$ and $R_8$ is nitro, cyano or $C_{1-3}$ alkylcarbonyl and the other is methoxy or amino optionally substituted by one or two $C_{1-6}$ alkyl or by $C_{2-7}$ alkanoyl; or $R_7$ is hydrogen and $R_8$ is $C_{1-6}$ alkyl or $C_{3-8}$ cycloalkyl; and
p represents zero or an integer 2.

3. A compound according to claim 1 or claim 2, wherein when $R^3$ represents hydroxy, alkoxy or acyloxy and $R^4$ is hydrogen, then the moiety $R_6$.N.CX.$R_5$ is trans to $R_3$.

4. A compound according to any one of claims 1 to 3, wherein the moiety $R_5$.N.CX.$R_6$ represents either pyrrolidonyl or piperidonyl.

5. A compound according to any one of claims 1 to 4, wherein $R_7$ represents hydrogen and $R_8$ represents methyl.

6. A compound according to any one of claims 1 to 5, wherein p represents zero.

7. A compound according to claim 1, selected from the group consisting of:
trans-3,4-dihydro-4-(2-oxopiperidin-1-yl)-2,2,6-trimethyl-2H-1-benzothiapyran-3-ol;
4-(2-oxopyrrolidin-1-yl)-2,2,6-trimethyl-2H-1-benzothiapyran-1,1-dioxide; and
trans-3,4-dihydro-4-(2-oxopiperidin-1-yl)-2,2,6-trimethyl-2H-1-benzothiapyran-3-ol-1,1-dioxide; or a pharmaceutically acceptable salt thereof, or a pharmaceutically acceptable solvate thereof.

8. A process for the preparation of a compound of formula (I) or, where appropriate a pharmaceutically acceptable salt thereof, or a pharmaceutically acceptable solvate thereof, which comprises;

i) treating a compound of formula (II):

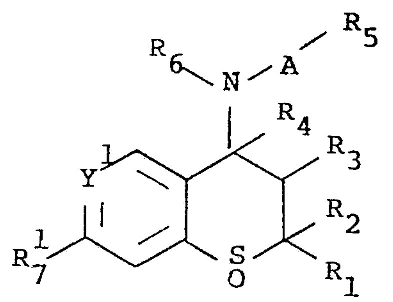

(II)

wherein $R_1$, $R_2$, $R_3$, $R_4$, $R_5$, $R_6$ and X are as defined in relation to formula (I), $R_7{}^1$ represents $R_7$ or a group or atom convertible to $R_7$ and $Y^1$ represents Y or a group convertible to Y,

with an appropriate redox reagent; or

ii) acylating a compound of formula (III):

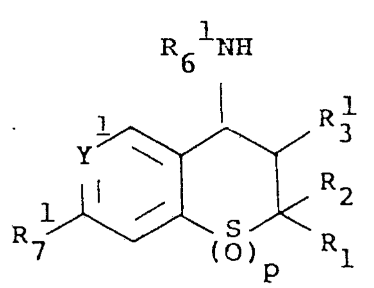

(III)

wherein, $R_1$, $R_2$, $R_7{}^1$ and $Y^1$ are as defined in relation to formula (II), $R_3{}^1$ represents hydroxy, alkoxy or acyloxy, $R_6{}^1$ is hydrogen or alkyl, and p represents zero or an integer 2,

a) with an acylating agent of formula (IV):

$R_{10}$-CO-$L_1$ (IV)

wherein $L_1$ is a leaving group, and $R_{10}$ is hydrogen, substituted or unsubstituted alkyl wherein the substituents are as hereinbefore defined for $R_5$; amino optionally substituted as hereinbefore defined for $R_5$; alkenyl; or optionally substituted aryl or heteroaryl as hereinbefore defined for $R_5$, or a group convertible to $R_5$ as hereinbefore defined, and thereafter, when $R_6$ is hydrogen and $R_{10}$ is $X^1(CH_2)_z$, where z is 3 or 4; and $X^1$ is a leaving group, cyclising the resultant compound;

b) with a compound of formula (V)

$X{=}C{=}N.R_{11}$ (V)

wherein $R_{11}$ is hydrogen, alkyl, alkenyl, alkanoyl optionally substituted by up to three halo atoms, or

phenyl optionally substituted by alkyl, alkoxy or halogen; and X is oxygen or sulphur, and thereafter when $R_{11}$ is hydrogen, optionally converting $R_{11}$; or

iii) for compounds of formula (I) wherein $R_5$ and $R_6$ together represent a linking chain of formula $-A^1-A^2$ as defined above in relation to formula (I) and wherein p represents 2, by reacting a compound of formula (VI):

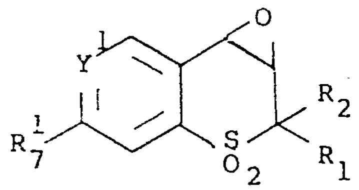

(VI)

wherein $R_1$, $R_2$ and p are as hereinbefore defined in relation to formula (I) and $R_7{}^1$ and $Y^1$ are as defined in relation to formula (III), with a compound of formula (VII):

$R_{13}NHCOR_{12}$ (VII)

wherein $R_{12}$ and $R_{13}$ together represent a linking chain $-A^1-A^2-$; or

iv) for compounds of formula (I) wherein A represents a bond and $R_5$ and $R_6$ together with the nitrogen to which they are attached form the above defined unsaturated heterocyclic ring, by reacting a compound of formula (VI) as defined above with an activated form of a compound of formula (VIIA):

$R_6'NHR_5'$ (VIIA)

wherein $R_5'$ and $R_6'$ form an unsaturated heterocyclic ring as defined above in respect of the variables $R_5$ and $R_6$;

and thereafter if required, carrying out one or more of the following optional steps:

(i) converting a compound of formula (I) into a further compound of formula (I);

(ii) converting $Y^1$ to Y;

(iii) converting $R_7{}^1$ to $R_7$;

(iv) forming a pharmaceutically acceptable salt of the compound of formula (I); or

(v) forming a pharmaceutically acceptable solvate of the compound of formula (I) or a pharmaceutically acceptable salt thereof.

9. A pharmaceutical composition which comprises a compound of formula (I), or a pharmaceutically acceptable salt thereof or a pharmaceutically acceptable solvate thereof, and a pharmaceutically acceptable carrier.

10. A compound of formula (I) or a pharmaceutically acceptable salt thereof, or a pharmaceutically acceptable solvate thereof, for use in the treatment of respiratory tract disorders or hypertension or disorders associated with smooth muscle contraction of the gastro intestinal tract or the abovementioned cardiovascular disorders.

11. The use of a compound of formula (I), or a pharmaceutically acceptable salt thereof, or a pharmaceutically acceptable solvate thereof for the manufacture of a medicament for the treatment of respiratory tract disorders or hypertension or disorders associated with smooth muscle contraction of the gastro intestinal tract or the abovementioned cardiovascular disorders.